(19) Europäisches Patentamt / European Patent Office / Office européen des brevets

(11) **EP 3 188 592 B1**

(12) **EUROPEAN PATENT SPECIFICATION**

(45) Date of publication and mention
of the grant of the patent:
**23.10.2019 Bulletin 2019/43**

(21) Application number: **15770979.1**

(22) Date of filing: **02.09.2015**

(51) Int Cl.:
*A01K 29/00* *(2006.01)*        *A61B 5/11* *(2006.01)*
*G01P 15/08* *(2006.01)*        *G01P 15/18* *(2013.01)*

(86) International application number:
**PCT/SE2015/050924**

(87) International publication number:
**WO 2016/036303 (10.03.2016 Gazette 2016/10)**

(54) **ARRANGEMENT AND METHOD FOR MEASURING RUMINATION OF AN ANIMAL**

ANORDNUNG UND VERFAHREN ZUR MESSUNG DES WIEDERKAUENS EINES TIERES

AGENCEMENT ET PROCÉDÉ PERMETTANT DE MESURER LA RUMINATION D'UN ANIMAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.09.2014 SE 1451033**

(43) Date of publication of application:
**12.07.2017 Bulletin 2017/28**

(73) Proprietor: **DeLaval Holding AB**
**147 21 Tumba (SE)**

(72) Inventor: **SCHAGERSTRÖM, Johan**
**147 21 Tumba (SE)**

(74) Representative: **Lilliehorn, Tobias**
**DeLaval International AB**
**Intellectual Property Support**
**Legal Affairs**
**P.O.Box 39**
**147 21 Tumba (SE)**

(56) References cited:
WO-A1-2014/199362        US-A- 6 104 294
US-A1- 2013 138 389        US-A1- 2014 163 406

## Description

## TECHNICAL FIELD

The technical field relates to arrangements and methods for measuring rumination of animals.

## RELATED ART

A trend in modern dairy farming is reduced workload and increased efficiency and profitability. Daily ruminating, eating, and activity patterns are closely related to health, feed consumption, and productivity of individual animals. Physiological and behavioral changes or abnormalities should be detected as early as possible to maintain good health and high milk production among the animals. High activity is used as a heat indicator and is used frequently in breeding programs.

Activity meters tracking the activity of each animal have been used since long. Some examples thereof are disclosed e.g. in US 6,104,294, US2014/163406 and US2013/138389, of which US2014/163406 mentions rumination analysis.

Eating and rumination sensors are also known in the art. One example thereof is the Cow-manager SensOor system commercially available from Agis Automatisering BV, Harmelen, the Netherlands. The sensor in the Cow-manager SensOor system is a molded microchip that has to be clicked into an adapted ear identification tag. A three-dimensional accelerometer continuously registers the movements of the cow's ear. Data is sent through a wireless connection via routers and coordinators, to a computer. Raw data is continuously collected and is classified each minute as one of the four behavioral categories "ruminating", "eating", "resting", and "active" with a proprietary model, which is subsequently expressed as percentage of behavior per hour as well as per day.

## SUMMARY

It is an aim of this document to reveal novel arrangements and methods for measuring rumination of individual animals, which are reliable, accurate, precise, and suitable to be implemented on commercial farms for animal management.

It is a further aim to reveal such novel arrangements and methods, which can also be used for measuring activity and feed consumption of individual animals, wherein the measured rumination, activity, and feed consumption can be used in feeding, health, and breeding management.

A first aspect refers to an arrangement for measuring rumination of an animal comprising a sensor and a processing device, as defined in claim 1. The sensor comprises an accelerometer for repeatedly sensing acceleration and is configured to be attached to a portion of the animal, wherein the movement of the sensor is at least partly correlated to the movement of the jaws of the animal during rumination e.g. in a similar fashion as the ear-attached sensor of the Cow-manager SensOor system is operating.

However, the processing device, which is operatively connected to the accelerometer to obtain the repeatedly sensed acceleration as an acceleration signal, is configured (i) to process the acceleration signal, thereby removing a DC component from the acceleration signal, (ii) to repeatedly determine a size of the processed acceleration signal in a selected frequency band, (iii) to determine a first threshold value, and at each time the size of the processed repeatedly sensed acceleration in the selected frequency band has been determined, (iv) to compare the last determined size of the processed acceleration signal in the selected frequency band and the first threshold value, and (v) to determine that the animal is ruminating if a rumination condition is met, wherein the rumination condition comprises that the last determined size of the processed acceleration signal in the selected frequency band is higher than the first threshold value, wherein the processing device is configured, at each time the size of the processed repeatedly sensed acceleration in the selected frequency band has been determined, to repeatedly determine a size of the processed acceleration signal outside the selected frequency band and to determine the first threshold value based on, e.g. to be equal to, the size of the processed acceleration signal outside the selected frequency band.

The above arrangement has proved to be reliable, accurate, precise, and suitable to be implemented on commercial farms for animal management.

In one embodiment, the acceleration signal is originating from acceleration measurements in one direction only. In such instance, the processing device may be configured to repeatedly Fourier transform the processed acceleration signal, preferably using the FFT, to repeatedly obtain a frequency spectrum of the processed acceleration signal, and to repeatedly determine the size of the processed acceleration signal in the selected frequency band from the frequency spectra.

In another embodiment, the accelerometer may be provided for repeatedly sensing the acceleration in at least two orthogonal directions, wherein the acceleration signal has at least two components. The processing device may then be configured to process the acceleration signal to obtain at least two processed signals, from which a respective DC component is removed, one signal for each of the at least two components, to repeatedly Fourier transform the at least two processed signals, preferably using the FFT, to repeatedly obtain at least two frequency spectra of the processed acceleration signal, to repeatedly combine the at least two frequency spectra of the processed acceleration signal to form a combined frequency spectrum, e.g. by means of selecting the strongest signal of the at least two spectra at each frequency of the frequency spectra, and to repeatedly determine the size of the processed acceleration signal in the selected frequency band from the combined frequency spectra.

In yet another embodiment, the accelerometer may be provided for repeatedly sensing the acceleration in three orthogonal directions, wherein the acceleration signal has three components, and the processing device may be configured (i) to process the acceleration signal to obtain three processed signals, from which a respective DC component is removed, one signal for each of the three components, (ii) to form an additional signal based on the three processed signals, (iii) to repeatedly Fourier transform the three processed signals and the additional signal, preferably using the FFT, to repeatedly obtain four frequency spectra, (iv) to repeatedly combine the four frequency spectra to form a combined frequency spectrum, e.g. by means of selecting the strongest signal of the four frequency spectra at each frequency of the frequency spectra, and (v) to repeatedly determine the size of the processed acceleration signal in the selected frequency band from the combined frequency spectra. The additional signal may be the G vector $V_L = (x^2 + y^2 + z^2)^{1/2} - 1$, where x, y, z are the three processed signals.

The latter two embodiments may be more reliable since acceleration due to rumination is detected in more than one direction.

In any of the above embodiments, the processing device may be configured to repeatedly determine the size of the processed acceleration signal in the selected frequency band as a size indicative of the intensity of the frequency spectra or combined frequency spectra within the selected frequency band. Since the size of the processed acceleration signal outside the selected frequency band is used in the measuring of the rumination of the animal, the processing device is configured to repeatedly determine the size of the processed acceleration signal outside the selected frequency band as a size indicative of the intensity of the frequency spectra or combined frequency spectra outside the selected frequency band.

Alternatively, the processing device may be configured to repeatedly determine the size of the processed acceleration signal in the selected frequency band as the maximum signal of the frequency spectra or combined frequency spectra within the selected frequency band. Since the size of the processed acceleration signal outside the selected frequency band is used in the measuring of the rumination of the animal, the processing device is configured to repeatedly determine the size of the processed acceleration signal outside the selected frequency band as the maximum signal of the frequency spectra or combined frequency spectra outside the selected frequency band.

In still another embodiment, the processing device may be configured to band pass filter the processed acceleration signal, to form a signal indicative of the absolute value of the band pass filtered processed acceleration signal, and to repeatedly determine the size of the processed acceleration signal in the selected frequency band from the signal indicative of the absolute value of the band pass filtered processed acceleration signal. Since the size of the processed acceleration signal outside the selected frequency band is used in the measuring of the rumination of the animal, the processing device may be configured to band-stop filter the processed acceleration signal, to form a signal indicative of the absolute value of the band-stop filtered processed acceleration signal, and to repeatedly determine the size of the processed acceleration signal outside the selected frequency band from the signal indicative of the absolute value of the band-stop filtered processed acceleration signal. The band stopping or band rejection may be performed by any kind of filter arrangement, and the lowest frequencies of the processed acceleration signal may be stopped or rejected as well.

In yet another embodiment, the accelerometer may be provided for repeatedly sensing the acceleration in at least two orthogonal directions, wherein the acceleration signal has at least two components. The processing device may then be configured (i) to process the acceleration signal to obtain at least two processed signals, from which a respective DC component has been removed, one signal for each of the at least two components, (ii) to band pass filter the at least two processed signals, (iii) to form signals indicative of the absolute values of the band pass filtered at least two processed signals, (iv) to combine the signals indicative of the absolute values of the band pass filtered at least two processed signals, and (v) to repeatedly determine the size of the processed acceleration signal in the selected frequency band from the combined signals. The combination may be made as indicated above for the Fourier transform approach.

Since the size of the processed acceleration signal outside the selected frequency band is used in the measuring of the rumination of the animal, the processing device may be configured to band-stop filter the at least two processed signals, to form signals indicative of the absolute values of the band-stop filtered at least two processed signals, to combine the signals indicative of the absolute values of the band-stop filtered at least two processed signals, and to repeatedly determine the size of the processed acceleration signal outside the selected frequency band from the combined signals indicative of the absolute values of the band-stop filtered at least two processed signals.

The selected frequency band may be in the range of about 1-4 Hz, and more preferably in the range of about 2-3 Hz, and most preferably at around 2.5 Hz. Alternatively, or additionally, the selected frequency band may comprise a first overtone of a frequency of the rumination of an animal.

A second aspect, not according to the invention, refers to an arrangement for measuring rumination of an animal comprising a sensor comprising an accelerometer for repeatedly sensing acceleration and configured to be attached to a portion of the animal, the movement of which being at least partly correlated to the movement of the jaws of the animal during rumination; and a processing device operatively connected to the accelerometer to obtain the repeatedly sensed acceleration as an acceleration signal. The processing device may be configured to process the acceleration signal, thereby removing a DC component from the acceleration signal, to repeatedly search the processed acceleration signal for a characteristic pattern, and at each time the processed acceleration signal has been searched, to determine that the animal is ruminating if the characteristic pattern is found the last time the processed acceleration signal was searched, wherein the characteristic pattern comprises repeated minimas of the processed acceleration signal.

The minimas may have each an amplitude, which is lower than a first threshold, which may be related to the average of the processed acceleration signal.

Further, the characteristic pattern may comprise that the average of the processed acceleration signal is above a second threshold and below a third threshold, which is higher than the second threshold.

Still further, the characteristic pattern may comprise that a variation from an average of the processed acceleration signal, such as e.g. variance, standard deviation, or absolute value of difference from the average, is below a fourth threshold, at least at a time before each of the minimas and/or at the minimas.

The time separation between the minimas may be in the range of about 20 to 300 s, and more preferably in the range of about 20 to 180 s, and most preferably in the range of about 30 to 100 s.

The minimas may have each a duration in the range of about 0.5-20 s, and more preferably in the range of about 1-8 s.

If the accelerometer is provided for repeatedly sensing the acceleration in two or three orthogonal directions, the acceleration signal has two or three components. The processing device may be configured to process the acceleration signal to obtain two or three processed signals, from which a respective DC component has been removed, one signal for each of the two or three components. Optionally, if three processed signals are available, an additional signal, e.g. the G vector $V_L = (x^2 + y^2 + z^2)^{1/2} - 1$, where x, y, z are the three processed signals may be formed.

The processing device may further be configured to combine the two or three processed signals and the optional additional signal, e.g. by summing them, to repeatedly search the combined signal for the characteristic pattern, and at each time the combined signal has been searched, to determine that the animal is ruminating if the characteristic pattern is found the last time the combined signal was searched, wherein the characteristic pattern comprises repeated minimas of the combined signal.

In the arrangement of the first and/or second aspect, the sensor may be configured to be attached to the neck of the animal or to a collar worn by the animal around the neck. The sensor and the processing device may be arranged together or remote from one another. The signals from the sensor may be transmitted to the processing device via wire or wirelessly.

Alternatively, the arrangement is provided as a unitary device, wherein the sensor and the processing device are mounted on a common support or in a common casing, wherein the entire arrangement may be configured to be attached to the neck of the animal or to a collar worn by the animal around the neck and may comprise a wireless transmitter operatively connected to the processing device and a battery for powering the sensor, the processing device, and the wireless transmitter.

In such event, the processing device may be configured to calculate rumination times based on the repeated determinations of whether the animal is ruminating and to log the rumination times; and the wireless transmitter may be configured to transmit the logged rumination times wirelessly to an external receiver.

A third aspect, according to the invention, refers to a method for measuring rumination of an animal wherein an acceleration is repeatedly sensed by a sensor comprising an accelerometer and being attached to a portion of the animal, the movement of which being at least partly correlated to the movement of the jaws of the animal during rumination. The acceleration signal is processed, thereby removing a DC component from the acceleration signal, a size of the processed acceleration signal in a selected frequency band is repeatedly determined, a first threshold value is determined, and at each time the size of the processed repeatedly sensed acceleration in the selected frequency band has been determined, the last determined size of the processed acceleration signal in the selected frequency band and the first threshold value are compared, and it is determined that the animal is ruminating if a rumination condition is met, wherein the rumination condition comprises that the last determined size of the processed acceleration signal in the selected frequency band is higher than the first threshold value, wherein the processing device is configured, at each time the size of the processed repeatedly sensed acceleration in the selected frequency band has been determined, to repeatedly determine a size of the processed acceleration signal outside the selected frequency band and to determine the first threshold value based on, e.g. to be equal to, the size of the processed acceleration signal outside the selected frequency band.

A fourth aspect, not according to the invention, refers to a method for measuring rumination of an animal wherein an acceleration is repeatedly sensed by a sensor comprising an accelerometer and being attached to a portion of the animal, the movement of which being at least partly correlated to the movement of the jaws of the animal during rumination. The acceleration signal is processed, thereby removing a DC component from the acceleration signal, the processed acceleration signal is repeatedly searched for a characteristic pattern, and at each time the processed acceleration signal has been searched, it is determined that the animal is ruminating if the characteristic pattern is found the last time the processed acceleration signal was searched, wherein the characteristic pattern comprises repeated minimas of the processed acceleration signal.

Further characteristics and advantages will be evident from the detailed description of embodiments given hereinafter, and the accompanying Figs. 1-8, which are given by way of illustration only.

## BRIEF DESCRIPTION OF THE DRAWINGS

Fig. 1 illustrates, schematically, an embodiment of the arrangement for measuring rumination of an animal and attached to a collar as worn by the animal around the neck.

Fig. 2 illustrates, schematically, in a block diagram the arrangement for measuring rumination of an animal.

Figs. 3-5 illustrate, schematically, in diagrams Fourier transforms as used by embodiments of the arrangement for measuring rumination of an animal.

Figs. 6-7 illustrate, schematically, in diagrams filtered signals as used by embodiments of the arrangement for measuring rumination of an animal.

Fig. 8 illustrates, schematically, in a diagram the absolute value of a high-pass filtered acceleration signal during rumination as used by an embodiment of the arrangement for measuring rumination of an animal.

## DETAILED DESCRIPTION OF EMBODIMENTS

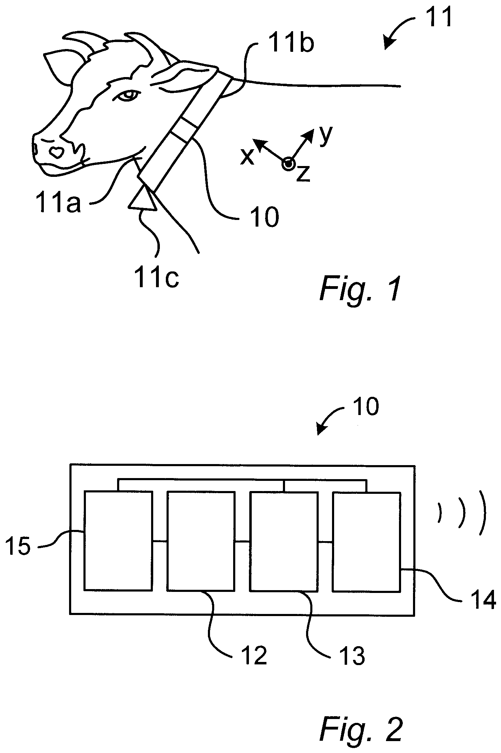

Fig. 1 illustrates, schematically, an embodiment of the arrangement 10 for measuring rumination of an animal 11 and attached to neck of the animal 11, e.g. by means of being attached to a collar 11b as worn by the animal 11 around the neck 11a thereof. The collar 11b may be provided with a weight 11c in a lower end thereof to ensure a correct positioning of the sensor 12. Fig. 2 illustrates, schematically, in a block diagram the arrangement 10 and its components.

The arrangement 10 comprises a sensor 12 including an accelerometer for repeatedly sensing acceleration. The attachment of the sensor 12 to the neck 11a of the animal 11 ensures that the movement of the accelerator is correlated at least partly to the movement of the jaws of the animal 11 during rumination.

A Cartesian coordinate system is indicated, wherein the X axis is parallel with the longitudinal direction of the neck of the animal 11, the Y axis is parallel with the main extension of the collar 11, and the Z axis is perpendicular to the surface of the animal (at the position of the sensor 12).

Alternatively, the sensor 12 is attached to another portion of the animal having a movement which is at least partly correlated to the movement of the jaws of the animal 11 during rumination.

The arrangement further comprises a processing device 13 operatively connected to the accelerometer to obtain the repeatedly sensed acceleration as an acceleration signal, a wireless transmitter 14 operatively connected to the processing device 13, and a battery 15 for powering the sensor 12, the processing device 13, and the wireless transmitter 14.

The processing device 13 is configured to process the acceleration signal, thereby removing a DC component from the acceleration signal (e.g by means of filtration), to repeatedly determine a size of the processed acceleration signal in a selected frequency band, to determine a threshold value, and at each time the size of the processed repeatedly sensed acceleration in the selected frequency band has been determined, to compare the last determined size of the processed acceleration signal in the selected frequency band and the threshold value, and to determine that the animal is ruminating if a rumination condition is met, wherein the rumination condition comprises that the last determined size of the processed acceleration signal in the selected frequency band is higher than the threshold value. The threshold value will be further discussed below.

Advantageously, the processing device 13 may be configured to calculate rumination times based on the repeated determinations of that the animal 11 is ruminating and to log the rumination times, and the wireless transmitter 14 may be configured to transmit the logged rumination times wirelessly to an external receiver 16 of e.g. an animal herd management system, a feeding management system, a health management system, and/or a breeding management system.

The selected frequency band may be in the range of about 1-4 Hz, and more preferably in the range of about 2-3 Hz, and most preferably at around 2.5 Hz and/or the selected frequency band may comprise a first overtone of a frequency of the rumination of an animal.

In one embodiment, the accelerometer is provided for repeatedly sensing the acceleration in three orthogonal directions (X, Y, Z), wherein the acceleration signal has three components. The processing device 13 is configured (i) to process the acceleration signal to obtain three processed signals, from which a respective DC component has been removed, one signal for each of the three components, (ii) to form an additional signal based on the three processed signals, (iii) to repeatedly Fourier transform the three processed signals and the additional signal, preferably using the FFT, to repeatedly obtain four frequency spectra, (iv) to repeatedly combine the four frequency spectra to form a combined frequency spectrum, e.g. by means of selecting the strongest signal of the four frequency spectra at each frequency of the frequency spectra, and (v) to repeatedly determine the size of the processed acceleration signal in the selected frequency band from the combined frequency spectra.

The additional signal may be the G vector

$$V_L = (x^2 + y^2 + z^2)^{1/2} - 1$$

where x, y, z are the three processed signals.

The measurements and calculations illustrated in the figures have been performed for an animal ruminating slightly more than thirty minutes.

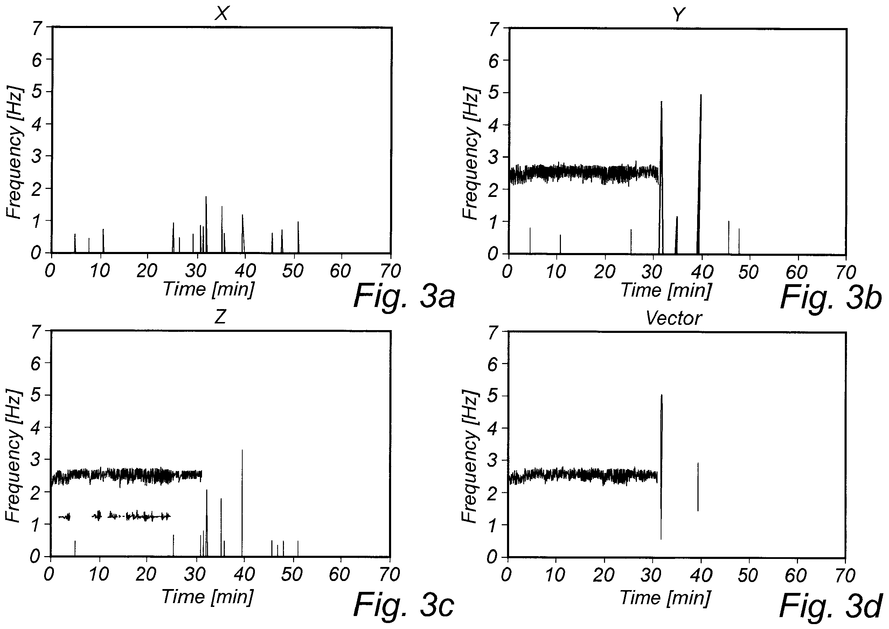

Fig. 3a (upper left Figure) illustrates, schematically, in a diagram the Fourier transform of the processed signal for the X axis, Fig. 3b (upper right Figure) illustrates, schematically, in a diagram the Fourier transform of the processed signal for the Y axis, Fig. 3c (lower left Figure) illustrates, schematically, in a diagram the Fourier transform of the processed signal for the Z axis, and Fig. 3d (lower right Figure) illustrates, schematically, in a diagram the Fourier transform of the G vector described above. Dark color indicates low signal value and light color indicates high signal value.

It can be seen that the Fourier transform shows strong signals at around 2.2-2.4 Hz during rumination for the Y axis, the Z axis, and the G vector. It can be seen that the strong signals in this frequency band terminate after slightly more than thirty minutes, when the animal stops ruminating. Thus, the Fourier transforms for the Y axis, Z axis, and/or G vector (including all three axes) could be used to determine whether the animal ruminates. It is believed that the strong signals at around 2.2-2.4 Hz is an overtone and that the signals at the fundamental frequency at half the above frequency are visible for the Y axis and Z axis.

If only one of the Fourier transforms for the Y axis, Z axis, and G vector is used, a rumination period could possibly be missed. This is remedied by the combination of the Fourier transforms disclosed above.

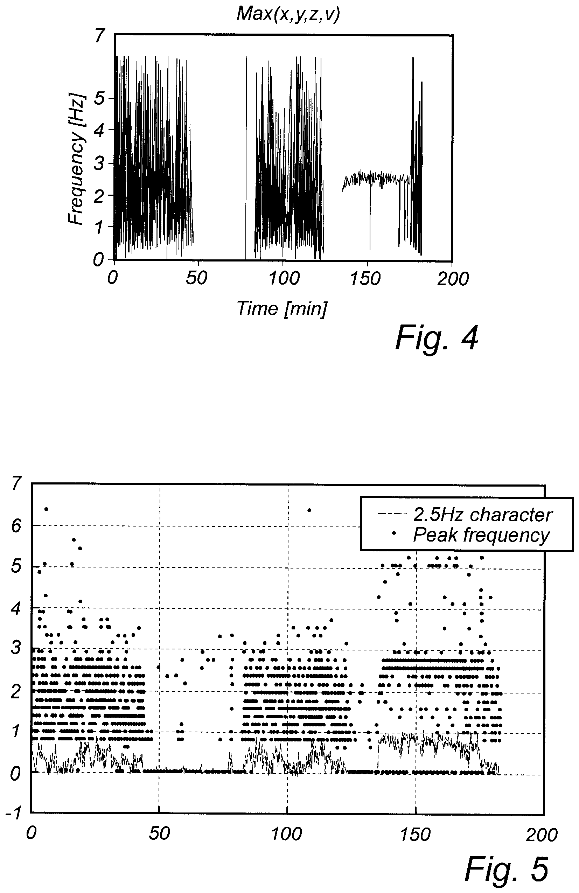

Fig. 4 illustrates, schematically, in a diagram such combined Fourier transform, wherein at each frequency the strongest signal of the four Fourier transforms is used. The animal is here eating from the beginning and about 45 minutes, resting between about 45 and 80 minutes, eating again between about 80 and 120 minutes, resting between about 120 to 135 minutes, ruminating between about 135 and 175 minutes. This can easily be observed in Fig. 4. During eating the frequency content of the combined Fourier transforms is covering a very broad frequency band, during resting no signals are obtained, and during rumination the frequency content of the combined Fourier transforms is covering a very narrow frequency band situated between 2 and 3 Hz. It is quite obvious that the best identification of rumination is obtained if the signals of the Fourier transform at a certain frequency (the rumination identification frequency) are compared with the signals at other frequencies.

Thus, in one embodiment the Fourier transform is analyzed to find peak frequencies at each time, and to calculate a variable $f_{2.5}[n]$ indicating whether the strongest signal $f_{max}$ at each instant has a frequency in the interval 2-3 Hz. The variable $f_{2.5}[n]$ becomes

$$f_{2.5}[n] = (f_{max}[n] > 2) \wedge (f_{max}[n] < 3)$$

The variable $f_{2.5}[n]$ is then low pass filtered with a very low cutoff frequency, and the result is shown in Fig. 5. The dots correspond to the peak frequencies at each instance and the solid line is the variable $f_{2.5}[n]$. If the variable $f_{2.5}[n]$ is close to one for a given time period, the animal is considered to be ruminating, when the variable $f_{2.5}[n]$ is varying a lot, the animal is considered to be eating, and when the variable $f_{2.5}[n]$ is close to zero for a given time period, the animal is considered to be resting. The condition for rumination may also encompass that a size of the Fourier transform in the interval 2-3 Hz is above a threshold, which may be a constant.

The processing device 13 is configured, at each time the size of the processed repeatedly sensed acceleration in the selected frequency band has been determined, to repeatedly determine a size of the processed acceleration signal outside the selected frequency band and to determine the threshold value based on the size of the processed acceleration signal outside the selected frequency band. The threshold may be a constant times the size of the processed acceleration signal outside the selected frequency band.

The rumination condition may also comprise that the last determined size of the processed acceleration signal in the selected frequency band is higher than a further threshold value, which is a constant.

In another embodiment, the processing device 13 may be configured to band pass the processed acceleration signal and to determine the size of the processed acceleration signal in the selected frequency band based on the band passed repeatedly sensed acceleration.

The accelerometer may be provided for repeatedly sensing the acceleration in at least two orthogonal directions Y, Z, wherein the acceleration signal has at least two components. The processing device 13 may be configured (i) to process the acceleration signal to obtain at least two processed signals, from which a respective DC component has been removed, one signal for each of the at least two components, (ii) to band pass filter the at least two processed signals, (iii) to form signals indicative of the absolute values of the band pass filtered at least two processed signals, (iv) to combine the signals indicative of the absolute values of the band pass filtered at least two processed signals, and (v) to repeatedly determine the size of the processed acceleration signal in the selected frequency band from the combined signals.

The processing device may further be configured to band-stop filter the at least two processed signals, to form signals indicative of the absolute values of the band-stop filtered at least two processed signals, to combine the signals indicative of the absolute values of the band-stop filtered at least two processed signals, and to repeatedly determine the size of the processed acceleration signal outside the selected frequency band from the combined signals indicative of the absolute values of the band-stop filtered at least two processed signals.

The rumination condition may here comprise that the last determined size of the processed acceleration signal in the selected frequency band is higher than a threshold value, which is related to the size of the processed acceleration signal outside the selected frequency band. The threshold value may be a constant times the size of the processed acceleration signal outside the selected frequency band.

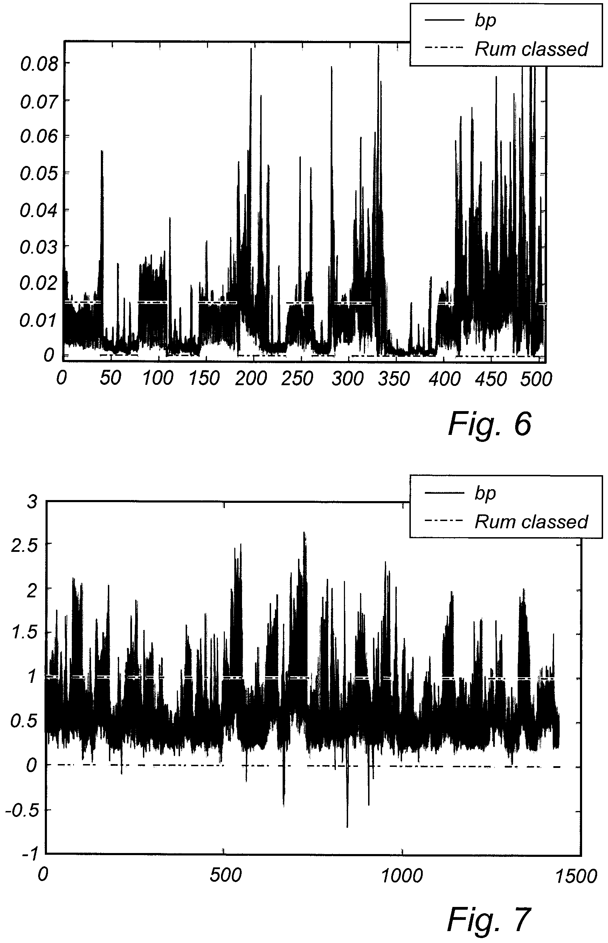

Fig. 6 illustrates, schematically, in a diagram a filtered signal for measuring rumination of an animal. The filter used is a band pass filter with cutoff frequencies at 2 and 3 Hz, allowing signals at frequencies between 2 and 3 Hz, but also signal spikes, to pass through. The horizontal lines indicate rumination (upper lines) and resting (lower lines).

It could sometimes be hard to distinguish rumination from resting (or eating) since the signals contain noise. To improve the situation, one or two other signals may be used: a band pass filtered signal between 0.5 and 2 Hz and a high pass filtered signal from 3 Hz and up. The signal at 2-3 Hz as shown in Fig. 6 may then be divided by the signal at 0.5-2 Hz, with the signal above 3 Hz, or with the sum of the signal at 0.5-2 Hz and the signal above 3 Hz. The latter one is illustrated in Fig. 7, i.e. the signal at 2-3 Hz divided by the sum of the signal at 0.5-2 Hz and the signal above 3 Hz. As before, the horizontal lines indicate rumination (upper lines) and resting (lower lines). As can be noted, rumination may in this signal be easier to distinguish from other activities.

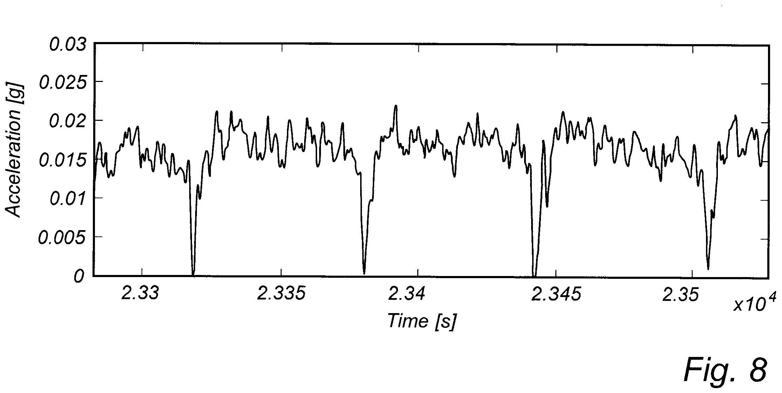

Fig. 8 illustrates, schematically, in a diagram a processed acceleration signal, which is correlated to the absolute value of a high-pass filtered acceleration signal during rumination as used by an embodiment of the arrangement for measuring rumination of an animal.

From the diagram, it can be seen that the animal is ruminating by means of bolus detection. When an animal ruminates, rumen content is formed as a bolus that is regurgitated and chewed a number of times. The four characteristic repeated drops correspond to short periods (e.g. 1-5 s) of bolus (or pause) wherein the yaws of the animal are at rest while a bolus is regurgitated, which occur before each longer period (e.g. 40-85 s) of chewing. The characteristic bolus and chewing pattern forms the rumination.

The arrangement, not according to the invention, for measuring rumination of an animal may here comprise similar structural components as described above, but the processing device 13 is instead configured to process the acceleration signal, thereby removing a DC component from the acceleration signal, to repeatedly search the processed acceleration signal for a characteristic pattern, and at each time the processed acceleration signal has been searched, to determine that the animal is ruminating if the characteristic pattern is found the last time the processed acceleration signal was searched, wherein the characteristic pattern comprises repeated minimas of the processed acceleration signal.

The characteristic pattern may comprise further features.

The minimas may each have an amplitude which is lower than a first threshold, which may be related to the average of the processed acceleration signal.

The characteristic pattern may comprise that the average of the processed acceleration signal is above a second threshold and below a third threshold, which is higher than the second threshold.

The characteristic pattern may comprise that a variation from an average of the processed acceleration signal, such as e.g. variance, standard deviation, or absolute value of difference from the average, is below a fourth threshold at least at a time, e.g. 10 seconds, before each of the minimas and/or at the minimas.

The time separation between the minimas may be in the range of about 20 to 300 s, and more preferably in the range of about 20 to 180 s, and most preferably in the range of about 30 to 100 s.

Further, the minimas may have each a duration in the range of about 0.5-20 s, and more preferably in the range of about 1-8 s.

The arrangement for measuring rumination of an animal is not restricted to the embodiment described in the figures, but may be varied freely within the scope of the claims. In particular, various features of the different embodiments may be combined in other none-disclosed combinations to form yet further embodiments within the scope of the claims. For example, instead of the signal processing taking place in a processing device connected to the sensor, the signal can be transferred unprocessed to a separate computer for processing.

**Claims**

1. An arrangement (10) for measuring rumination of an animal (11) comprising:

    - a sensor (12) comprising an accelerometer for repeatedly sensing an acceleration and configured to be attached to a portion (11a) of the animal, the movement of which being at least partly correlated to the movement of the jaws of the animal during rumination; and
    - a processing device (13) operatively connected to the accelerometer to obtain the repeatedly sensed acceleration as an acceleration signal, **characterized in that**

- the processing device is configured (i) to process the acceleration signal, thereby removing a DC component from the acceleration signal, (ii) to repeatedly determine a size of the processed acceleration signal in a selected frequency band, (iii) to determine a first threshold value, and at each time the size of the processed repeatedly sensed acceleration in the selected frequency band has been determined, (iv) to compare the last determined size of the processed acceleration signal in the selected frequency band and the first threshold value, and (v) to determine that the animal is ruminating if a rumination condition is met, wherein the rumination condition comprises that the last determined size of the processed acceleration signal in the selected frequency band is higher than the first threshold value, and
- wherein the processing device is configured, at each time the size of the processed repeatedly sensed acceleration in the selected frequency band has been determined, to repeatedly determine a size of the processed acceleration signal outside the selected frequency band and to determine the first threshold value based on, e.g. to be equal to, the size of the processed acceleration signal outside the selected frequency band.

2. The arrangement of claim 1 wherein the processing device is configured to repeatedly Fourier transform the processed acceleration signal, preferably using the FFT, to repeatedly obtain a frequency spectrum of the processed acceleration signal, and to repeatedly determine the size of the processed acceleration signal in the selected frequency band from the frequency spectra.

3. The arrangement of claim 1 wherein

- the accelerometer is provided for repeatedly sensing the acceleration in at least two orthogonal directions (Y, Z), wherein the acceleration signal has at least two components; and
- the processing device is configured to process the acceleration signal to obtain at least two processed signals, from which a respective DC component is removed, one signal for each of the at least two components, to repeatedly Fourier transform the at least two processed signals, preferably using the FFT, to repeatedly obtain at least two frequency spectra of the processed acceleration signal, to repeatedly combine the at least two frequency spectra of the processed acceleration signal to form a combined frequency spectrum, e.g. by means of selecting the strongest signal of the at least two spectra at each frequency of the frequency spectra, and to repeatedly determine the size of the processed acceleration signal in the selected frequency band from the combined frequency spectra.

4. The arrangement of any of claims 2-3 wherein the processing device is configured to repeatedly determine the size of the processed acceleration signal in the selected frequency band as a size indicative of the intensity of the frequency spectra or combined frequency spectra within the selected frequency band.

5. The arrangement of claim 4 wherein the processing device is configured to repeatedly determine the size of the processed acceleration signal outside the selected frequency band as a size indicative of the intensity of the frequency spectra or combined frequency spectra outside the selected frequency band.

6. The arrangement of any of claims 2-3 wherein the processing device is configured to repeatedly determine the size of the processed acceleration signal in the selected frequency band as the maximum signal of the frequency spectra or combined frequency spectra within the selected frequency band.

7. The arrangement of claim 6 wherein the processing device is configured to repeatedly determine the size of the processed acceleration signal outside the selected frequency band as the maximum signal of the frequency spectra or combined frequency spectra outside the selected frequency band.

8. The arrangement of claim 1 wherein the processing device is configured to band pass filter the processed acceleration signal, to form a signal indicative of the absolute value of the band pass filtered processed acceleration signal, and to repeatedly determine the size of the processed acceleration signal in the selected frequency band from the signal indicative of the absolute value of the band pass filtered processed acceleration signal.

9. The arrangement of claim 8 wherein the processing device is configured to band-stop filter the processed acceleration signal, to form a signal indicative of the absolute value of the band-stop filtered processed acceleration signal, and to repeatedly determine the size of the processed acceleration signal outside the selected frequency band from the signal indicative of the absolute value of the band-stop filtered processed acceleration signal.

10. The arrangement of claim 1 wherein

- the accelerometer is provided for repeatedly sensing the acceleration in at least two orthogonal directions (Y, Z), wherein the acceleration signal has at least two components; and
- the processing device is configured (i) to process the acceleration signal to obtain at least two processed signals, from which a respective DC component has been removed, one signal for each of the at least two components, (ii) to band pass filter the at least two processed signals, (iii) to form signals indicative of the absolute values of the band pass filtered at least two processed signals, (iv) to combine the signals indicative of the absolute values of the band pass filtered at least two processed signals, and (v) to repeatedly determine the size of the processed acceleration signal in the selected frequency band from the combined signals.

11. The arrangement of claim 10 wherein the processing device is configured to band-stop filter the at least two processed signals, to form signals indicative of the absolute values of the band-stop filtered at least two processed signals, to combine the signals indicative of the absolute values of the band-stop filtered at least two processed signals, and to repeatedly determine the size of the processed acceleration signal outside the selected frequency band from the combined signals indicative of the absolute values of the band-stop filtered at least two processed signals.

12. The arrangement of any of claims 1-11 wherein the selected frequency band is in the range of about 1-4 Hz, and more preferably in the range of about 2-3 Hz, and most preferably at around 2.5 Hz.

13. The arrangement of any of claims 1-11 wherein the selected frequency band comprises a first overtone of a frequency of the rumination of an animal.

14. A method for measuring rumination of an animal (11) comprising:

- repeatedly sensing an acceleration by a sensor (12) comprising an accelerometer and being attached to a portion (11a) of the animal, the movement of which being at least partly correlated to the movement of the jaws of the animal during rumination, **characterized in that**
- the acceleration signal is processed, thereby removing a DC component from the acceleration signal;
- a size of the processed acceleration signal in a selected frequency band is repeatedly determined;
- a first threshold value is determined; and
- at each time the size of the processed repeatedly sensed acceleration in the selected frequency band has been determined, the last determined size of the processed acceleration signal in the selected frequency band and the first threshold value are compared, and it is determined that the animal is ruminating if a rumination condition is met, wherein the rumination condition comprises that the last determined size of the processed acceleration signal in the selected frequency band is higher than the first threshold value, and wherein, at each time the size of the processed repeatedly sensed acceleration in the selected frequency band has been determined, a size of the processed acceleration signal outside the selected frequency band is repeatedly determined, and wherein the first threshold value is determined based on the size of the processed acceleration signal outside the selected frequency band.

15. The method of claim 14 wherein the processed acceleration signal is band pass filtered; a signal indicative of the absolute value of the band pass filtered processed acceleration signal is formed; and the size of the processed acceleration signal in the selected frequency band is repeatedly determined from the signal indicative of the absolute value of the band pass filtered processed acceleration signal.

**Patentansprüche**

1. Anordnung (10) zum Messen eines Wiederkäuens eines Tiers (11), Folgendes umfassend:

- einen Sensor (12), umfassend einen Beschleunigungsmesser zum wiederholten Erfassen einer Beschleunigung, und konfiguriert, um an einem Abschnitt (11a) des Tiers befestigt zu werden, wobei dessen Bewegung wenigstens teilweise mit der Bewegung der Kiefer des Tiers während des Wiederkäuens korreliert; und
- eine Verarbeitungsvorrichtung (13), die mit dem Beschleunigungsmesser wirkverbunden ist, um die wiederholt erfasste Beschleunigung als ein Beschleunigungssignal zu erhalten, **dadurch gekennzeichnet, dass**
- die Verarbeitungsvorrichtung konfiguriert ist, (i) um das Beschleunigungssignal zu verarbeiten, wobei dadurch eine Gleichstromkomponente aus dem Beschleunigungssignal entfernt wird, (ii) um wiederholt eine Größe des verarbeiteten Beschleunigungssignals in einem ausgewählten Frequenzband zu bestimmen, (iii) um einen ersten Schwellenwert zu bestimmen, und jedes Mal, wenn die Größe der verarbeiteten, wiederholt erfassten Beschleunigung in dem ausgewählten Frequenzband bestimmt worden ist, (iv) um die zuletzt bestimmte Größe des verarbeiteten Beschleunigungssignals in dem ausgewählten Frequenzband und den ersten Schwellenwert zu vergleichen, und (v) um zu bestimmen, dass das Tier wiederkäut, wenn eine Wiederkäuungsbedingung erfüllt ist, wobei die Wiederkäuungsbedingung umfasst, dass die zuletzt bestimmte Größe des verarbeiteten Beschleunigungssignals in dem ausgewählten Frequenzband höher ist als der erste Schwellenwert, und

- wobei die Verarbeitungsvorrichtung konfiguriert ist, um, jedes Mal, wenn die Größe der verarbeiteten wiederholt erfassten Beschleunigung in dem ausgewählten Frequenzband bestimmt worden ist, eine Größe des verarbeiteten Beschleunigungssignals außerhalb des ausgewählten Frequenzbands wiederholt zu bestimmen, und um den ersten Schwellenwert basierend auf, z. B. um dieser zu entsprechen, der Größe des verarbeiteten Beschleunigungssignals außerhalb des ausgewählten Frequenzbands zu bestimmen.

2. Anordnung nach Anspruch 1, wobei die Verarbeitungseinrichtung konfiguriert ist, um das verarbeitete Beschleunigungssignal wiederholt nach Fourier zu transformieren, bevorzugt unter Verwendung der FFT, um wiederholt ein Frequenzspektrum des verarbeiteten Beschleunigungssignals zu erhalten, und um wiederholt die Größe des verarbeiteten Beschleunigungssignals in dem gewählten Frequenzband aus den Frequenzspektren zu bestimmen.

3. Anordnung nach Anspruch 1, wobei

- der Beschleunigungsmesser zum wiederholten Erfassen der Beschleunigung in wenigstens zwei orthogonalen Richtungen (Y, Z) bereitgestellt ist, wobei das Beschleunigungssignal wenigstens zwei Komponenten aufweist; und
- die Verarbeitungsvorrichtung konfiguriert ist, um das Beschleunigungssignal zu verarbeiten, um wenigstens zwei verarbeitete Signale zu erhalten, von denen eine jeweilige Gleichstromkomponente entfernt wird, ein Signal für jede der wenigstens zwei Komponenten, um die wenigstens zwei verarbeiteten Signale wiederholt nach Fourier zu transformieren, bevorzugt unter Verwendung der FFT, um wiederholt wenigstens zwei Frequenzspektren des verarbeiteten Beschleunigungssignals zu erhalten, um wiederholt die wenigstens zwei Frequenzspektren des verarbeiteten Beschleunigungssignals zu kombinieren, um ein kombiniertes Frequenzspektrum auszubilden, z. B. mittels eines Auswählens des stärksten Signals der wenigstens zwei Spektren bei jeder Frequenz der Frequenzspektren, und um wiederholt die Größe des verarbeiteten Beschleunigungssignals in dem ausgewählten Frequenzband aus den kombinierten Frequenzspektren zu bestimmen.

4. Anordnung nach einem der Ansprüche 2-3, wobei die Verarbeitungsvorrichtung konfiguriert ist, um wiederholt die Größe des verarbeiteten Beschleunigungssignals in dem ausgewählten Frequenzband als eine die Intensität der Frequenzspektren oder der kombinierten Frequenzspektren anzeigende Größe innerhalb des ausgewählten Frequenzbands zu bestimmen.

5. Anordnung nach Anspruch 4, wobei die Verarbeitungsvorrichtung konfiguriert ist, um wiederholt die Größe des verarbeiteten Beschleunigungssignals außerhalb des ausgewählten Frequenzbands als eine die Intensität der Frequenzspektren oder der kombinierten Frequenzspektren anzeigende Größe außerhalb des ausgewählten Frequenzbands zu bestimmen.

6. Anordnung nach einem der Ansprüche 2-3, wobei die Verarbeitungsvorrichtung konfiguriert ist, um wiederholt die Größe des verarbeiteten Beschleunigungssignals in dem ausgewählten Frequenzband als das maximale Signal der Frequenzspektren oder der kombinierten Frequenzspektren innerhalb des ausgewählten Frequenzbands zu bestimmen.

7. Anordnung nach Anspruch 6, wobei die Verarbeitungsvorrichtung konfiguriert ist, um wiederholt die Größe des verarbeiteten Beschleunigungssignals außerhalb des ausgewählten Frequenzbands als das maximale Signal der Frequenzspektren oder der kombinierten Frequenzspektren außerhalb des ausgewählten Frequenzbands zu bestimmen.

8. Anordnung nach Anspruch 1, wobei die Verarbeitungsvorrichtung konfiguriert ist, um das verarbeitete Beschleunigungssignal durch einen Bandpass zu filtern, um ein den Absolutwert des durch einen Bandpass gefilterten verarbeiteten Beschleunigungssignals anzeigendes Signal auszubilden, und um wiederholt die Größe des verarbeiteten Beschleunigungssignals in dem ausgewählten Frequenzband aus dem den Absolutwert des durch einen Bandpass gefilterten verarbeiteten Beschleunigungssignals anzeigenden Signal zu bestimmen.

9. Anordnung nach Anspruch 8, wobei die Verarbeitungsvorrichtung konfiguriert ist, um das verarbeitete Beschleunigungssignal durch eine Bandsperre zu filtern, um ein den Absolutwert des durch eine Bandsperre gefilterten verarbeiteten Beschleunigungssignals anzeigendes Signal auszubilden, und um wiederholt die Größe des verarbeiteten Beschleunigungssignals außerhalb des ausgewählten Frequenzbands aus dem den Absolutwert des durch eine Bandsperre gefilterten verarbeiteten Beschleunigungssignals anzeigenden Signal zu bestimmen.

10. Anordnung nach Anspruch 1, wobei

- der Beschleunigungsmesser zum wiederholten Erfassen der Beschleunigung in wenigstens zwei orthogonalen Richtungen (Y, Z) bereitgestellt ist, wobei das Beschleunigungssignal wenigstens zwei Komponenten aufweist; und
- die Verarbeitungsvorrichtung konfiguriert ist, (i) um das Beschleunigungssignal zu verarbeiten, um wenigstens zwei verarbeitete Signale zu erhalten, von denen eine jeweilige Gleichstromkomponente entfernt wurde, ein Signal für jede der wenigstens zwei Komponenten, (ii) um die wenigstens zwei verarbeiteten Signale durch einen Bandpass zu filtern, (iii) um die Absolutwerte der durch einen Bandpass gefilterten wenigstens zwei verarbeiteten Signale anzeigende Signale auszubilden, (iv) um die die Absolutwerte der durch einen Bandpass gefilterten wenigstens zwei verarbeiteten Signale anzeigenden Signale zu kombinieren, und (v) um wiederholt die Größe des verarbeiteten Beschleunigungssignals in dem ausgewählten Frequenzband aus den kombinierten Signalen zu bestimmen.

11. Anordnung nach Anspruch 10, wobei die Verarbeitungsvorrichtung konfiguriert ist, um die wenigstens zwei verarbeiteten Signale durch eine Bandsperre zu filtern, um den Absolutwert der durch eine Bandsperre gefilterten wenigstens zwei verarbeiteten Beschleunigungssignale anzeigende Signale auszubilden, um die die Absolutwerte der durch eine Bandsperre gefilterten wenigstens zwei verarbeiteten Signale anzeigenden Signale zu kombinieren, und um wiederholt die Größe des verarbeiteten Beschleunigungssignals außerhalb des ausgewählten Frequenzbands aus den die Absolutwerte der durch eine Bandsperre gefilterten wenigstens zwei verarbeiteten Signale anzeigenden kombinierten Signalen zu bestimmen.

12. Anordnung nach einem der Ansprüche 1-11, wobei das ausgewählte Frequenzband in dem Bereich von etwa 1-4 Hz, und stärker bevorzugt in dem Bereich von etwa 2-3 Hz, und am stärksten bevorzugt bei etwa 2,5 Hz liegt.

13. Anordnung nach einem der Ansprüche 1-11, wobei das ausgewählte Frequenzband einen ersten Oberton einer Frequenz des Wiederkäuens eines Tiers umfasst.

14. Verfahren zum Messen des Wiederkäuens eines Tiers (11), Folgendes umfassend:

- wiederholtes Erfassen einer Beschleunigung durch einen Sensor (12), umfassend einen Beschleunigungsmesser und befestigt an einem Abschnitt (11a) des Tiers, wobei dessen Bewegung wenigstens teilweise mit der Bewegung der Kiefer des Tiers während des Wiederkäuens korreliert, **dadurch gekennzeichnet, dass**
- das Beschleunigungssignal verarbeitet wird, wobei dadurch eine Gleichstromkomponente aus dem Beschleunigungssignal entfernt wird;
- eine Größe des verarbeiteten Beschleunigungssignals in einem ausgewählten Frequenzband wiederholt bestimmt wird;
- ein erster Schwellenwert bestimmt wird; und
- jedes Mal, wenn die Größe der verarbeiteten wiederholt erfassten Beschleunigung in dem ausgewählten Frequenzband bestimmt worden ist, die zuletzt bestimmte Größe des verarbeiteten Beschleunigungssignals in dem ausgewählten Frequenzband und der erste Schwellenwert verglichen werden, und bestimmt wird, dass das Tier wiederkäut, wenn eine Wiederkäuungsbedingung erfüllt ist, wobei die Wiederkäuungsbedingung umfasst, dass die zuletzt bestimmte Größe des verarbeiteten Beschleunigungssignals in dem ausgewählten Frequenzband höher ist als der erste Schwellenwert, und wobei jedes Mal, wenn die Größe der verarbeiteten wiederholt erfassten Beschleunigung in dem ausgewählten Frequenzband bestimmt worden ist, eine Größe des verarbeiteten Beschleunigungssignals außerhalb des ausgewählten Frequenzbands wiederholt bestimmt wird, und wobei der erste Schwellenwert basierend auf der Größe des verarbeiteten Beschleunigungssignals außerhalb des ausgewählten Frequenzbands bestimmt wird.

15. Verfahren nach Anspruch 14, wobei das verarbeitete Beschleunigungssignal durch einen Bandpass gefiltert wird;

ein den Absolutwert des durch einen Bandpass gefilterten verarbeiteten Beschleunigungssignals anzeigendes Signal ausgebildet wird; und die Größe des verarbeiteten Beschleunigungssignals in dem ausgewählten Frequenzband wiederholt aus dem den Absolutwert des durch einen Bandpass gefilterten verarbeiteten Beschleunigungssignals anzeigenden Signal bestimmt wird.

## Revendications

1. Agencement (10) permettant de mesurer la rumination d'un animal (11), comprenant:

- un capteur (12) comprenant un accéléromètre pour détecter de manière répétée une accélération et configuré pour être fixé à une partie (11a) de l'animal, le mouvement de celui-ci étant au moins partiellement corrélé au mouvement des mâchoires de l'animal pendant la rumination ; et

- un dispositif de traitement (13) connecté de manière fonctionnelle à l'accéléromètre afin d'obtenir l'accélération détectée de manière répétée en tant que signal d'accélération, **caractérisé en ce que**

- le dispositif de traitement est configuré (i) pour traiter le signal d'accélération, éliminant ainsi un composant CC du signal d'accélération (ii) pour déterminer de manière répétée une taille du signal d'accélération traité dans une bande de fréquences sélectionnée (iii) pour déterminer une première valeur seuil, et à chaque fois que la taille de l'accélération traitée détectée de manière répétée dans la bande de fréquences sélectionnée a été déterminée (iv) pour comparer la dernière taille déterminée du signal d'accélération traité dans la bande de fréquences sélectionnée et la première valeur seuil, et (v) pour déterminer que l'animal rumine si une condition de rumination est remplie, la condition de rumination comprenant le fait que la dernière taille déterminée du signal d'accélération traité dans la bande de fréquences sélectionnée est supérieure à la première valeur seuil, et

- dans lequel le dispositif de traitement est configuré, à chaque fois que la taille de l'accélération traitée détectée de manière répétée dans la bande de fréquences sélectionnée a été déterminée, pour déterminer de manière répétée une taille du signal d'accélération traité en dehors de la bande de fréquences sélectionnée, et pour déterminer la première valeur seuil sur la base de, par exemple de manière à être égale à, la taille du signal d'accélération traité, en dehors de la bande de fréquences sélectionnée.

2. Agencement selon la revendication 1, dans lequel le dispositif de traitement est configuré pour appliquer de manière répétée la transformée de Fourier au signal d'accélération traité, de préférence à l'aide de la TFE, pour obtenir de manière répétée un spectre de fréquences du signal d'accélération traité, et pour déterminer de manière répétée la taille du signal d'accélération traité dans la bande de fréquences sélectionnée à partir des spectres de fréquences.

3. Agencement selon la revendication 1, dans lequel

- l'accéléromètre est utilisé pour détecter de manière répétée l'accélération dans au moins deux directions orthogonales (Y, Z), le signal d'accélération ayant au moins deux composants ; et

- le dispositif de traitement est configuré pour traiter le signal d'accélération afin d'obtenir au moins deux signaux traités, à partir desquels un composant CC respectif est retiré, un signal pour chacun desdits deux composants, pour appliquer de manière répétée la transformée de Fourier auxdits signaux traités, de préférence à l'aide de la TFR, pour obtenir de manière répétée au moins deux spectres de fréquences du signal d'accélération traité, pour combiner de manière répétée lesdits deux spectres de fréquences du signal d'accélération traité pour former un spectre de fréquences combiné, par exemple en sélectionnant le signal le plus puissant desdits deux spectres à chaque fréquence des spectres de fréquences, et pour déterminer de manière répétée la taille du signal d'accélération traité dans la bande de fréquences sélectionnée à partir des spectres de fréquences combinés.

4. Agencement selon l'une quelconque des revendications 2 et 3, dans lequel le dispositif de traitement est configuré pour déterminer de manière répétée la taille du signal d'accélération traité dans la bande de fréquences sélectionnée en tant qu'une taille indiquant l'intensité des spectres de fréquences ou des spectres de fréquences combinés à l'intérieur de la bande de fréquences sélectionnée.

5. Agencement selon la revendication 4, dans lequel le dispositif de traitement est configuré pour déterminer de manière répétée la taille du signal d'accélération traité en dehors de la bande de fréquences sélectionnée en tant qu'une taille indiquant l'intensité des spectres de fréquences ou des spectres de fréquences combinés en dehors de la bande de fréquences sélectionnée.

6. Agencement selon l'une quelconque des revendications 2 à 3, dans lequel le dispositif de traitement est configuré pour déterminer de manière répétée la taille du signal d'accélération traité dans la bande de fréquences sélectionnée en tant que signal maximum des spectres de fréquences ou des spectres de fréquences combinés à l'intérieur de la bande de fréquences sélectionnée.

7. Agencement selon la revendication 6, dans lequel le dispositif de traitement est configuré pour déterminer de manière répétée la taille du signal d'accélération traité en dehors de la bande de fréquences sélectionnée en tant que signal maximum des spectres de fréquences ou des spectres de fréquences combinés en dehors de la bande de fréquences sélectionnée.

8. Agencement selon la revendication 1, dans lequel le dispositif de traitement est configuré pour filtrer par filtre passe-bande le signal d'accélération traité, pour former un signal indiquant la valeur absolue du signal d'accélération traité filtré par filtre passe-bande, et pour déterminer de manière répétée la taille du signal d'accélération traité dans la bande de fréquences sélectionnée à partir du signal indiquant la valeur absolue du signal d'accélération traité filtré par filtre passe-bande.

9. Agencement selon la revendication 8, dans lequel le dispositif de traitement est configuré pour filtrer par filtre coupe-bande le signal d'accélération traité, pour former un signal indiquant la valeur absolue du signal d'accélération traité filtré par filtre coupe-bande, et pour déterminer de manière répétée la taille du signal d'accélération traité en dehors de la bande de fréquences sélectionnée à partir du signal indiquant la valeur absolue du signal d'accélération traité filtré par filtre coupe-bande.

10. Agencement selon la revendication 1, dans lequel

   - l'accéléromètre est utilisé pour détecter de manière répétée l'accélération dans au moins deux directions orthogonales (Y, Z), le signal d'accélération ayant au moins deux composants ; et
   - le dispositif de traitement est configuré (i) pour traiter le signal d'accélération afin d'obtenir au moins deux signaux traités, à partir desquels un composant CC a été retiré, un signal pour chacun desdits deux composants (ii) pour filtrer par filtre passe-bande lesdits deux signaux traités (iii) pour former des signaux indiquant les valeurs absolues desdits deux signaux traités filtrés par filtre passe-bande (iv) pour combiner les signaux indiquant les valeurs absolues desdits deux signaux traités filtrés par filtre passe-bande, et (v) pour déterminer de manière répétée la taille du signal d'accélération traité dans la bande de fréquences sélectionnée à partir des signaux combinés.

11. Agencement selon la revendication 10, dans lequel le dispositif de traitement est configuré pour filtrer par filtre coupe-bande lesdits deux signaux traités, pour former des signaux indiquant les valeurs absolues desdits deux signaux traités filtrés par filtre coupe-bande, pour combiner les signaux indiquant les valeurs absolues desdits deux signaux traités filtrés par filtre coupe-bande, et pour déterminer de manière répétée la taille du signal d'accélération traité en dehors de la bande de fréquences sélectionnée à partir des signaux combinés indiquant les valeurs absolues desdits deux signaux traités filtrés par filtre coupe-bande.

12. Agencement selon l'une quelconque des revendications 1 à 11, dans lequel la bande de fréquences sélectionnée est dans la plage d'environ 1 à 4 Hz, et plus préférablement dans la plage d'environ 2 à 3 Hz et idéalement à environ 2,5 Hz.

13. Agencement selon l'une quelconque des revendications 1 à 11, dans lequel la bande de fréquences sélectionnée comprend un premier harmonique d'une fréquence de la rumination d'un animal.

14. Procédé permettant de mesurer la rumination d'un animal (11), comprenant :

   - la détection de manière répétée d'une accélération par un capteur (12) comprenant un accéléromètre et étant fixé à une partie (11a) de l'animal, le mouvement de celui-ci au étant au moins partiellement corrélé au mouvement des mâchoires de l'animal pendant la rumination, **caractérisé en ce que**
   - le signal d'accélération est traité, éliminant ainsi un composant CC du signal d'accélération ;
   - une taille du signal d'accélération traité dans une bande de fréquences sélectionnée est déterminée de manière répétée ;
   - une première valeur seuil est déterminée ; et
   - à chaque fois que la taille de l'accélération traitée détectée de manière répétée dans la bande de fréquences sélectionnée a été déterminée, la dernière taille déterminée du signal d'accélération traité dans la bande de fréquences sélectionnée et la première valeur seuil sont comparées, et il est déterminé que l'animal rumine si une condition de rumination est remplie, la condition de rumination étant que la dernière taille déterminée du signal d'accélération traité dans la bande de fréquences sélectionnée soit supérieure à la première valeur seuil, et, à chaque fois que la taille de l'accélération traitée détectée de manière répétée dans la bande de fréquences sélectionnée a été déterminée, une taille du signal d'accélération traité en dehors de la bande de fréquences sélectionnée étant déterminée de manière répétée, et la première valeur seuil étant déterminée sur la base de la taille du signal d'accélération traité en dehors de la bande de fréquences sélectionnée.

15. Procédé selon la revendication 14, dans lequel le signal d'accélération traité est filtré par filtre passe-bande ; un signal indiquant la valeur absolue du signal d'accélération traité et filtré par filtre passe-bande est formé ; et la taille du signal d'accélération traité, dans la bande de fréquences sélectionnée, est déterminée de manière répétée à partir du signal indiquant la valeur absolue du signal d'accélération traité filtré par filtre passe-bande.

*Fig. 1*

*Fig. 2*

*Fig. 3a*

*Fig. 3b*

*Fig. 3c*

*Fig. 3d*

Max(x,y,z,v)

Fig. 4

Fig. 5

Fig. 6

Fig. 7

*Fig. 8*

**EP 3 188 592 B1**

## REFERENCES CITED IN THE DESCRIPTION

**Patent documents cited in the description**

- US 6104294 A
- US 2014163406 A
- US 2013138389 A